# EUROPÄISCHE PATENTSCHRIFT

(11) **EP 1 275 376 B1**
(45) Veröffentlichungstag und Bekanntmachung des Hinweises auf die Patenterteilung: **19.04.2006**
(21) Anmeldenummer: 01124148.6
(22) Anmeldetag: 10.10.2001
(51) Int. Cl.: A61K 9/10, A61K 47/32, A61K 47/38, A61K 31/375, A61K 9/00, A61K 47/00

(54) **Zweiphasige, tropfbare Hydrogele zur Anwendung am Auge**
Two-phase hydrogel for application in drops at the eye
Hydrogel à deux phases pour l'application en gouttes à l'oeil

(30) Priorität: 06.07.2001 DE 10132876
(43) Veröffentlichungstag der Anmeldung: 15.01.2003
(73) Patentinhaber: MEDPROJECT PHARMA-ENTWICKLUNGS- UND VERTRIEBSGESELLSCHAFT mbH, 82054 Sauerlach (DE)
(72) Erfinder: Kreitmeier, Piroska, 81927 München (DE); Muggenthaler, Monika, 82541 Ammerland (DE); Polzer, Heinz, Dr., 88138 Weissensberg (DE)
(74) Vertreter: von Füner, Nicolai

(56) Entgegenhaltungen:
- WO-A-00/49990
- US-A- 5 252 318
- US-A- 5 296 228
- OECHSNER M ET AL: "Polyacrylic acid/polyvinylpyrrolidone bipolymeric systems. I. Rheological and mucoadhesive properties of formulations potentially useful for the treatment of dry-eye-syndrome" EUROPEAN JOURNAL OF PHARMACEUTICS AND BIOPHARMACEUTICS, ELSEVIER SCIENCE PUBLISHERS B.V., AMSTERDAM, NL, Bd. 47, Nr. 2, 1. März 1999 (1999-03-01), Seiten 113-118, XP004257050 ISSN: 0939-6411
- KUMAR S ET AL: "Modification of in situ gelling behavior of carbopol solutions by hydroxypropyl methylcellulose." JOURNAL OF PHARMACEUTICAL SCIENCES. UNITED STATES MAR 1995, Bd. 84, Nr. 3, März 1995 (1995-03), Seiten 344-348, XP001109576 ISSN: 0022-3549

## Beschreibung

Die Erfindung betrifft neue Arzneimittel, und zwar Emulsionen, die insbesondere zur Anwendung am Auge geeignet sind. Übliche Darreichungsformen für Augenmedikamente sind Salben, Tropfen, Inserts oder Gele, während Emulsionen, die eine ölige und eine wäßrige Phase enthalten, bisher kaum zum Einsatz kommen. Derartige Emulsionen können jedoch von besonderem Interesse für die Anwendung am Auge sein, denn der natürliche Tränenfilm enthält neben der wäßrigen Schicht ebenfalls einen kleinen Anteil an Lipid(Öl)phase (ca. 1 %), welche die Abgrenzung zur Umwelt bildet. Entsprechend erscheint die Anwendung von Emulsionen mit kleinem Lipidanteil als "naturähnliche Tränenersatzmittel" als geeignet. Über die Anwendung lipidhaltiger Augentropfen ist berichtet worden (Rieger G. Lipidcontaining eye drops. A step closer to natural tears. Ophthalmologica 1990;201:206-212). Diese lipidhaltigen Augentropfen wurden aus handelsüblichen Lipidemulsionen zur parenteralen Ernährung hergestellt. Die Phasen entmischen sich beim Stehen, können jedoch durch Schütteln wieder dispergiert werden.

Lipidemulsionen eignen sich ebenso als Arzneimittelträger, insbesondere für Arzneimittel, deren Wirkstoffe wasserunlöslich oder hydrolyseempfindlich sind, sich jedoch in einer öligen, am Auge verträglichen Substanz lösen (gelöste Stoffe werden in Augenpräparaten in der Regel gegenüber suspendierten, mikronisierten Feststoffen bevorzugt, weil die homogene Verteilung des Wirkstoffs stets konstante Dosierung aus dem Behältnis gewährleistet). Die Herstellung von Emulsionen ist in der pharmazeutischen und kosmetischen Industrie ein alltäglicher Prozeß, wobei verschiedene Techniken angewendet werden. Es ist jedoch schwierig, für die Anwendung am Auge geeignete Emulsionen herzustellen, weil die üblichen Emulgatoren am Auge nicht gut verträglich sind oder zu funktionellen Beeinträchtigungen führen, so daß sich ihr Einsatz für diesen Zweck verbietet. Wir haben festgestellt, daß für das Auge geeignete ölige Substanzen in Carbomerhaltigen Gelen permanent emulgiert werden können, d.h. es findet keine Phasentrennung beim Stehen statt, so daß die Anwendung ohne vorausgehende Schüttelprozedur erfolgen kann. Einige Patente über Emulsionen (WO 95/31211) und Gel-Emulsionen (DE-OS 195 11 322) existieren, die speziell für die Anwendung am Auge ausgearbeitet wurden. Sie sollen als Tränenersatzmittel und/oder als Arzneimittelträger verwendet werden.

Gele unter Verwendung von Polyacrylat (Carbomer) und Mischungen von Polyacrylat (Carbomer) mit anderen Polymeren sind bekannt. In kürzlich erschienenen Publikationen wird beschrieben, daß die Kombination von 0,2 % Polyacrylat mit z. B. 6 % Povidon günstigere Eigenschaften als Augenpräparat hat als reine Polyacrylatgele mit und ohne Konservierung. Die niedrigere scheinbare Viskosität ("Ausgangsviskosität") erleichtert die Handhabung bei der Anwendung am Auge, und die (erwünschte) Mucoadhäsion der Polymerkombinationen ist besser als in reinen Polyacrylatgelen (Oechsner M, Keipert S. Polyacrylic acid/polyvinylpyrrolidone bipolymeric systems. 1. Rheological and mucoadhesive properties of formulations potentially useful for the treatment of dry-eye-syndrome. Eur J Pharm Biopharm 1999;47:113-118; Oechsner M, Keipert S. Interactions between Carbopol 980 and additional 2^{nd} macromolecule in aqueous formulations. Proc 1^{st} World Meeting APGI/APV, Budapest 9/11 May 1995; 16-17). Die Kombination von 0,2-0,4 % Polyacrylat (Carbomer) mit 1-2 % Hydroxypropylmethylcellulose ergibt Produkte, die als Augengele und Wirkstoffträger brauchbar sind (Kumar S, Himmelstein KJ. Modification of in Situ Gelling Behavior of Carbopol Solutions by Hydroxypropyl Methylcellulose. J Pharm Sci 1995;3:344-348).

Ferner ist aus der DE-42 09 722 ein tropfbares Gel zur Anwendung am Auge bekannt, das eine Viskosität von 10 000 bis 50 000 mPa · s aufweist, was erreicht wird durch die Zusammensetzung, enthaltend
1) ein Polyacrylat in einer Menge von 0,05 bis 3% und
2) Polyvinylalkohol und/oder Dextran in einer Menge von 0,1 und 10% und
3) die für Gele zur Anwendung am Auge üblichen Hilfsstoffe und
4) Salze zur stabilen Einstellung der Viskosität,
wobei das Verhältnis von Polyvinylalkohol und/oder Dextran zu Polyacrylat zwischen 1:1 und 8:1 liegt.

Die DE 691 17 509 betrifft eine reversibel gelierende wäßrige flüssige pharmazeutische Zusammensetzung, enthaltend eine wäßrige flüssige Lösung mit einem Gehalt an 0,1-30 Gew.-% eines thermisch-empfindlich gelierenden Polymeren und 0,01-10 Gew.-% eines pH-empfindlich gelierenden Polymeren, wobei die wäßrige flüssige Zusammensetzung einen reversiblen Viskositätsänderungsbereich von 200 - 1 000 000 mPas in Reaktion auf gleichzeitige Veränderung sowohl der Temperatur als auch des pH-Werts über Bereiche von 0-60°C bzw. von pH-Wert 2,5 bis pH-Wert 7,4 aufweist. Gemäß der DE 691 17 509 kann als thermisch-empfindlich gelierendes Polymeres unter anderem Alkylcellulose oder Hydroxyalkylcellulose, als pH-empfindlich gelierendes Polymeres ein Polyacrylat eingesetzt werden. Die pharmazeutische Zusammensetzung gemäß DE 691 17 509 kann ferner ein Salz, Hilfsstoffe wie beispielsweise Mannit, Polyvinylalkohol, und eine Base enthalten. Die pharmazeutische Zusammensetzung gemäß DE 691 17 509 liegt in Form einer freifließenden Flüssigkeit oder von Tropfen vor, die unmittelbar nach der Verabreichung unter minimalen Nebenwirkungen der Gelbildung unterliegen.

Die DE 695 12 589 betrifft ein Mittel zur Behandlung von Erkrankungen, die durch Störungen der Talgdrüsen bei Menschen und Tieren verursacht werden, das eine Gelsalbe darstellt, umfassend in einem wäßrigen System eine die Talgdrüsensekretion inhibierende Menge eines entsprechenden Wirkstoffs, ein Niederalkanol, ein Carboxyvinylpolymer, mindestens einen mehrwertigen Alkohol, ausgewählt aus der Gruppe, bestehend aus Niederalkylenglycol mit zwei bis sechs Kohlenstoffatomen, Glycerin und Polyethylenglycol mit einem mittleren Molekulargewicht von 200 bis 2 000, mindestens ein Alkanolamin mit ein bis vier Kohlenstoffatomen, Dialkanolamin mit zwei bis acht Kohlenstoffatomen und Trialkanolamin mit drei bis zwölf Kohlenstoffatomen und mindestens ein filmbildendes Mittel, ausgewählt aus der Gruppe, bestehend aus Carboxymethylcellulose, Hydroxyethylcellulose und Polyvinylpyrrolidon und Wasser. Das Mittel gemäß DE 69512589 kann als Gel vorliegen (enthaltend Carbomer®940 und Hydroxyethylcellulose).

Die US 4 966 773 beschreibt ein Mittel zur Behandlung von trockenem Auge, enthaltend ein Retinoid und ein pharmazeutisch annehmbares ophthalmisches Bindemittel. Das Bindemittel kann in Form einer Salbe vorliegen Es können auch wäßrige Lösungen, enthaltend Methylcellulose, Polyvinylalkohol, Carbopol etc. oder Gele, basierend auf Carbopol, Cellulosederivaten und Kombinationen davon, eingesetzt werden. Die Zusammensetzung kann ferner Methylcellulose, Dextran, Polyvinylalkohol, Polyvinylpyrrolidon etc. enthalten.

Die DE 690 33 199 beschreibt eine Zusammensetzung zur Befeuchtung von Epithelzellen, die Wasser, eine befeuchtende Menge eines wasserquellbaren, jedoch wasserunlöslichen vernetzten bioadhäsiven Polycarbonsäure-Polymers und vorzugsweise ein konsistenzverbessemdes Mittel enthält. Als bevorzugtes bioadhäsives Polymer wird CARBOPOL®934 eingesetzt. Als konsistenzverbessemdes Mittel können Hydroxypropylcellulose, Hydroxyethyl-cellulose, Polyvinylpyrrolidon oder Polyvinylalkohol eingesetzt werden. Die wäßrige Zusammensetzung kann eine Konsistenz bei Raumtemperatur einer kaum gießbaren Flüssigkeit bis zu der eines Gels aufweisen. Die Zusammensetzung kann Öle und Emulgiermittel, z.B. Hydroxypropylmethylcellulose, Polyvinylalkohol und Povidon, enthalten. Die Emulgiermittel werden dabei dazu verwendet, Öl-in-Wasser-Emulsionen herzustellen. Die Zusammensetzung kann ein Polycarbophil CARBOPOL®EX55), GARBOPOL®934, ein hydriertes Palmölglycerid-Dispergiermittel, schweres Mineralöl, Glycerin, Methylparaben und Wasser enthalten. Diese Zusammensetzung stellt eine Emulsion dar und weist eine gelartige Konsistenz auf.

Die US 5 296 228 beschreibt u.a. eine eine pharmazeutische Verbindung freisetzende, eintropfbare, lang lagerfähige Zusammensetzung mit verzögerter Wirkstofffreisetzung (Depoteffekt), die folgende Bestandteile umfasst:
eine Vielzahl von porösen lonenaustauscherharzteilchen mit einem Teilchendurchmesser von ungefähr 1 µm bis ungefähr 50 µm, wobei diese lonenaustauscherharzteilchen in einer wässrigen Lösung aus etwa 0,1 Gew.-% bis etwa 10 Gew.-% Methylcellulose und etwa 0,01 Gew.-% bis etwa 10 Gew.-% Polycarboxylat inkorporiert sind, wobei diese wässrige Lösung eine Freifließ-Viskosität aufweist, die bei Raumtemperatur und einem pH-Wert zwischen etwa 2,5 und 4,0 das Einträufeln als Tropfen erlaubt, und bei einem pH-Wert von etwa 7,4 und einer Temperatur von etwa 35° C eine gelartige Viskosität besitzt;
mindestens eine innerhalb der genannten Poren der lonenaustauscherharzteilchen ionisch gebundene pharmazeutische Verbindung, wobei die porenförmigen lonaustauscherharzteilchen eine Porengröße aufweisen, die so klein ist, um eine Diffusion der Methylcellulose und des Polycarboxylats in die Poren zu verhindern.

Bei der Anwendung am Auge haben Carbomergele die Eigenschaft, ihre Konsistenz nach Applikation beträchtlich zu verändern. Damit ist ein erwünschter, wasserspendender Effekt verbunden, wodurch dieses Gel, insbesondere bei trockenem Auge, Vorteile hat. Um jedoch gleichzeitig eine gegenüber rein wässrigen Tropfen längere Verweildauer zu erzielen, muß die Carbomerkonzentration so hoch sein, daß das Gel relativ hochviskos und damit nur aus Tuben entnehmbar und deshalb unvorteilhaft bei der Anwendung ist. Durch eine bereits bekannte Technik (DE-PS 42 09 722) läßt sich die Viskosität in geeigneter Weise einstellen. Gele mit auf diese Weise eingestellter Viskosität sind eine geeignete Grundlage zur Herstellung stabiler Emulsionen öliger Substanzen als Augenmedikament.

Aufgabe der vorliegenden Erfindung war es, zweiphasige Hydrogele (Gel-Emulsionen), insbesondere zur Anwendung am Auge, zur Verfügung zu stellen, die folgende Eigenschaften besitzen:
□ Die Gel-Emulsionen sind tropfbar und aus handelsüblichen Augentropffläschchen entnehmbar.
□ Sie weisen eine einstellbare Restviskosität am Auge auf, die maßgeblich für Verträglichkeit und Verweildauer verantwortlich ist (s. DE-PS 42 09 722).
□ Alle üblicherweise in der Ophthalmologie verwendeten Wirkstoffe und Konservierungsmittel können in die Rezeptur inkorporiert werden, und zwar sowohl wasser- wie auch in der Ölphase lösliche Stoffe.

Die hier zusammengefaßten Eigenschaften werden nachfolgend detailliert beschrieben:
1) Die Gel-Emulsionen sind tropfbar: Für die Tropfbarkeit ist die "Ausgangsviskosität", die sich am Ende des Herstellungsvorgangs ergibt, ausschlaggebend. Die Tropfbarkeit gewährleistet eine bessere Handhabung, da das Produkt aus einer handelsüblichen Augentropfflasche angewendet werden kann. Die Dosierungsgenauigkeit ist aus Flaschen wesentlich besser als bei Gelen mit dem aus Tuben üblicherweise kommenden "Strang". Geeignete Ausgangsviskositäten liegen im Bereich von 1 000 - 50 000 mPas (Viskositätswerte beziehen sich auf die in den Beispielen aufgeführte Meßmethode).
2) Am Auge stellt sich durch die Vermischung der Gel-Emulsion mit Bestandteilen des Tränenfilmes, in erster Linie mit den anorganischen Salzen, eine bestimmte "Restviskosität" ein. Diese Restviskosität ist aufgrund der bekannten Eigenschaften des Carbomers (Gelstruktur bricht zusammen) erheblich geringer als die Ausgangsviskosität vor dem Kontakt mit der Tränenflüssigkeit.
   Die sich am Auge einstellende Restviskosität ist letztendlich ausschlaggebend für Verträglichkeit und Verweildauer der Gel-Emulsion auf der Augenoberfläche. Durch steigende Restviskosität werden ebenso Wirkungsdauer und Wirkungsintensität des Augenmedikamentes positiv beeinflußt. Eine zu hohe Restviskosität führt hingegen zu Klebeffekten und Fremdkörpergefühl, also zu Verträglichkeitsproblemen. Geeignete Restviskositäten liegen im Bereich von 15 - 2000 mPas (Viskositäten beziehen sich auf die in den Beispielen aufgeführten Meßmethoden), bevorzugt im Bereich von 15 - 50 mPas.
3) Der Einarbeitung von Ölphase oder Wirkstoffen und Konservierungsmitteln in Carbomergele sind einerseits dadurch Grenzen gesetzt, daß es für eine stabile Öl-in-Wasser-Emulsion eine Obergrenze für die Menge an Ölphase gibt, die sich in die Gelgrundlage einarbeiten läßt. Das Überschreiten dieser Grenze führt zum Ausscheiden der öligen Phase aus der Emulsion. Ein weiteres Problem ergibt sich daraus, dass der überwiegende Anteil der in der Ophthalmologie verwendeten Wirkstoffe aus organischen Basen oder deren Salzen besteht, weshalb es andererseits durch Interaktion mit den Carboxylgruppen des Carbomers gelegentlich zu Ausfällungen kommen kann. Auch einige wichtige Konservierungsmittel können diesen unerwünschten Effekt hervorrufen.

Die erfindungsgemäße Lösung für die Formulierung einer Gel-Emulsion mit den in den 3 Punkten genannten Eigenschaften läßt sich wie folgt beschreiben:

Um aus der Kombination der unter den Punkten 1) und 2) aufgeführten Eigenschaften eine sinnvolle und zweckmäßige Rezeptur zu erstellen, galt es, zwei sich gleichsinnig ändernde Effekte zu beeinflussen, nämlich Ausgangs- und Restviskosität, und sie darüber hinaus so zu gestalten, daß die Ölphase stabil darin emulgiert werden konnte.

Die Tropfbarkeit der Gel-Emulsion erfordert eine relativ niedrige Ausgangsviskosität und damit einen relativ geringen Materialeinsatz des Gelbildners. Daraus ergibt sich in der Regel nach Applikation am Auge auch eine niedrige Restviskosität, die dann kaum zu einer merklichen Verbesserung der Verweildauer der Gel-Emulsion am Auge führt. Erhöht man den Materialeinsatz des Gelbildners (Carbomer) zur Erzielung einer geeigneteren (höheren) Restviskosität, so resultiert daraus eine Ausgangsviskosität, die in der Praxis eine Tropfbarkeit aus Flaschen nicht mehr erlaubt. Durch gezielte Salzzugabe (anorganische Salze oder auch Salze organischer Amine und Aminosäuren) zu diesen Gelen mit hoher Ausgangsviskosität kann jedoch die Ausgangsviskosität reduziert werden bis zu einem Punkt, bei dem eine zufriedenstellende Tropfbarkeit erreicht wird.

Ein Bestandteil der Erfindung beinhaltet somit die Entwicklung einer Rezeptur, bei der ein definiertes Verhältnis von Ausgangs- und Restviskosität einstellbar ist, mit dem Ziel, eine tropfbare Gel-Emulsion mit verlängerter Verweildauer auf der Augenoberfläche herzustellen. Da jedoch zusätzlich die unter Punkt 3) genannte Einarbeitung von Wirkstoffen, Ölphase und ggf. Konservierungsmitteln mitberücksichtigt werden muß, konnte dies nicht allein durch Herausfinden einer geeigneten Carbomerkonzentration und anschließender Salzzugabe, also durch Ausnutzen bekannter Carbomereigenschaften, erreicht werden, sondem es mußte eine geeignete neue Formulierung gefunden werden.

Als geeignet hinsichtlich Formulierbarkeit und guter Verträglichkeit am Auge erwiesen sich Gemische von Polyacrylat/Polyvinylpyrrolidon (PVP), Polyacrylat/Dextran, Polyacrylat/Cellulose(derivat), insbesondere Hydroxypropylmethylcellulose (HPMC) oder Polyacrylat/ Polyvinylalkohol (PVA).

Erfindungsgemäß wird somit beansprucht:
1) Eine tropfbare Gel-Emulsion, insbesondere zur Anwendung am Auge, enthaltend ein Polymergemisch von Polyacrylat/Polyvinylalkohol, Polyacrylat/Polyvinylpyrrolidon, Polyacrylat/Dextran oder Polyacrylat/ Cellulosederivat, und eine Ölphase.
2) Die unter Punkt 1) genannte Gel-Emulsion kann Salze, insbesondere Natriumacetat zur Beeinflussung der Ausgangsviskosität enthalten.
3) Die unter Punkt 1) und 2) genannte Gel-Emulsion kann wirkstofffrei sein, oder auch Wirkstoffe enthalten. Diese können in der wäßrigen oder öligen Phase gelöst oder suspendiert sein. Als Wirkstoffe kommen alle in der Ophthalmologie üblicherweise eingesetzten Wirkstoffe in Betracht.
4) Die unter Punkt 1), 2) und 3) genannte Gel-Emulsion kann gegebenenfalls zusätzlich ein Konservierungsmittel enthalten, wobei hierfür alle in der Ophthalmologie üblicherweise eingesetzten Konservierungsstoffe in Betracht kommen.
5) Die unter Punkt 1), 2), 3) und 4) genannte Gel-Emulsion kann zusätzlich mit geeigneten Stoffen isotonisiert werden, wozu nichtionische Verbindungen, wie oben genannt, eingesetzt werden.
6) Zur Einstellung der Euhydrie und zur Ausbildung der Gelstruktur ist es erforderlich, zusätzlich mit einer Base zu neutralisieren. Als Basen sind Natronlauge, Amine oder auch Aminosäuren geeignet.
7) Ein Verfahren zur Herstellung der erfindungsgemäßen Gel-Emulsion, sowie deren Verwendung in der Ophthalmologie.

Überraschend und wider Erwarten stellte sich heraus, daß es schwierig ist, stabile Gele aus Polyacrylat (Carbomer), einem zweiten Polymer und der Ölphase herzustellen. Da Gele zur Anwendung am Auge steril sein müssen, ist die. Autoklavierbarkeit der Polymer-Suspensionen oder der Gelgrundlage zwingend notwendig. An die Temperaturstabilität dieser Gele sind daher besonders hohe Ansprüche zu stellen. Dies gilt natürlich auch bezüglich der erforderlichen Haltbarkeit des Fertigproduktes im Endbehältnis über die Produktlaufzeit. Erfindungsgemäß wurde gefunden, daß nicht jedes beliebige Mischungsverhältnis von Carbomer-Gelgrundlage und Ölphase zu in ihren allgemeinen Merkmalen stabilen Produkten führt (siehe Tabelle 1 und 2).

Erfindungsgemäß können als Polyacrylat alle marktüblichen Carbomertypen eingesetzt werden. Zweckmäßigerweise wählt man wasserlösliche Typen mit einem Molekulargewicht zwischen 1.000.000 und 4.000.000, im konkreten Fall waren dies Carbopol® 934, 934P, 940, 941, 951, 954, 971 P, 974, 974P, 980, 981 (siehe auch H.P. Fiedler, Lexikon der Hilfsstoffe für Pharmazie, Kosmetik und angrenzende Gebiete, 3. Auflage 1989, Edition Cantor Aulendorf, Stichworte Polyacrylsäure, Carbopol).

Üblicherweise wird das Polyacrylat in einer Menge von 0,05 - 3% (bezogen auf die Gesamtmenge der Emulsion), bevorzugt 0,05 - 1 %, eingesetzt.

Für PVP kommen alle handelsüblichen Povidon-Typen in Frage, bevorzugt jedoch K 25 und K 30.

Üblicherweise wird PVP in einer Menge von 0,05 - 10% (bezogen auf die Gesamtmenge der Emulsion), bevorzugt 1 - 7%, eingesetzt.

Bei PVA führen jedoch nur vollständig verseifte Typen (d.h. Hydrolysegrad von mindestens 99%, Molukelargewicht 15 000 - 200 000) im richtigen Mischungsverhältnis mit Carbomer zu viskositäts- und pH-stabilen Gelen.

Üblicherweise wird PVA in einer Menge von 0,1 - 10% (bezogen auf die Gesamtmenge der Emulsion), bevorzugt 0,3 - 1,5%, eingesetzt.

Dextran wird üblicherweise in einer Menge von 0,1 - 10% (bezogen auf die Gesamtmenge der Emulsion), bevorzugt 0,3 - 5%, HPMC in einer Menge von 0,05 - 3% (bezogen auf die Gesamtmenge der Emulsion), bevorzugt 0,1 - 2%, eingesetzt. Bevorzugt wird ein Polymergemisch von Polyacrylat/Povidon eingesetzt. Polyacrylat/PVA wird bevorzugt in einem Verhältnis im Bereich von 1:1 zu 1:8 eingesetzt.

Als Ölphase kommen pharmazeutisch oder kosmetisch verwendbare Neutralöle in Betracht, deren Augenverträglichkeit bereits bekannt ist, z. B.: Rizinusöl, mittelkettige Triglyceride (z. B. Miglyol, Myritol), Cetearyl Octanoate (z. B. Luvitol) oder ähnliche.

Zur Einstellung der Ausgangsviskosität werden Salze, insbesondere Natriumacetat, in einer Menge von bis zu 0,5% (bezogen auf die Gesamtmenge der Emulsion), bevorzugt bis zu 0,2%, eingesetzt.

Für die Bildung von Polyacrylatgelen ist der Zusatz von Basen erforderlich, so dass die erfindungsgemäß beanspruchten Gele ebenfalls Basen enthalten müssen. Prinzipiell ist die Gelbildung mit allen Basen möglich. Bevorzugt wird Trometamol als Base verwendet, daneben die basische Aminosäure Lysin. Es eignen sich auch andere basische Aminosäuren oder Amine. Für handelsübliche Augengele wird meist Natronlauge benutzt.

Da Gele zur Anwendung am Auge isoton sein sollten, müssen diese Gele mit geeigneten Mitteln isotonisiert werden. Das Auge toleriert Osmolaritäten zwischen 100 und 450 mOsmol/l. In der Regel werden Augentropfen mit einem lsotonans etwa auf 260 bis 320 mOsmol/l eingestellt, und zwar mit Salzen. Salze (lonen) können für Carbomer in den beanspruchten Konzentrationen aus den oben erwähnten Gründen nicht verwendet werden, weil sie die Gelstruktur zerstören. Es kommen hierfür nur nichtionische Verbindungen in Betracht. Deshalb werden für Carbomergele beispielsweise Sorbitol oder Mannitol (in einer Menge von 1,8 - 8,2%) oder Glycerol (insbesondere Glycerol 85% in einer Menge von 1 % - 4,9%) verwendet.

Die Gele können ohne pharmakologisch wirksamen Bestandteil als Tränensubstitutionsmittel eingesetzt werden. Andererseits sind sie auch als Vehikel für Wirkstoffe geeignet. Es ist schwierig, organische Basen (= Wirkstoffe) und Konservierungsmittel vom Quat-Typ in klare und stabile Polyacrylatgele einzuarbeiten. Andererseits eignet sich auch die Ölphase als Lösungsmittel für wasserunlösliche oder im Kontakt mit Wasser instabile Wirkstoffe (z. B. Hormone, Cyclosporin). Erfindungsgemäß wurden Formulierungen und Verfahren gefunden, die dies ermöglichen.

Ein Beispiel für ein übliches Konservierungsmittel ist Benzalkoniumchlorid (BAC), wobei zu beachten ist, daß der Zusatz eines Konservierungsmittels für Augenarzneimittel in Mehrdosenbehältern vorgeschrieben ist. Der Zusatz eines zweiten, gut wasserlöslichen Polymers verbessert die Kompatibilität von Benzalkoniumchlorid mit Carbomer (darüber hinaus ermöglicht er auch die Einarbeitung von Wirkstoffen in gelöster Form). Andere Konservierungsmittel sind mit den erfindungsgemäßen Kombinationen ebenfalls kompatibel, z.B. die am Auge gut verträglichen Konservierungsmittel Thiomersal, Chlorhexidingluconat/-acetat, Parabene. BAC gilt als repräsentativ für die Gruppe der "Quats", auch für polymere, Quat-ähnliche Stoffe.

Die erfindungsgemäßen Gel-Emulsionen werden nach dem folgenden Verfahren hergestellt:

Aus einer Polyacrylat-Dispersion in Wasser, welche die notwendigen Bestandteile zur Isotonisierung und ggf. Konservierung enthält (Mischung A), wird mit einer wäßrigen Lösung der Basen Lysin oder Trometamol und Natriumacetat (Mischung C) ein Gel hergestellt.

In der wäßrigen Lösung des zweiten zur Herstellung der Gel-Emulsionen erforderlichen Polymers (PVA, PVP, Dextran, HPMC), die ggf. das Konservierungsmittel oder einen Teil davon enthalten kann, wird die Ölphase mit einem Homogenisator feinst verteilt (Mischung B). Ehe wieder Entmischung der Phasen eintritt, wird Mischung B in das aus Mischung A und Mischung C gebildete Gel durch wirksames Rühren homogen eingearbeitet. Dies ergibt eine stabile Gel-Emulsion.

Zur Herstellung der zur Anwendung am Auge vorgesehenen Gel-Emulsionen ist eine aseptische Herstellung aus sterilisierten Mischungen A, B und C erforderlich. Die Mischungen A, B und C werden entweder durch Autoklavieren oder durch Membranfiltration sterilisiert, je nachdem, welche Methode für die Mischung oder ihre Bestandteile geeignet ist.

Die in der Gel-Emulsion insgesamt enthaltene Wassermenge wird zur Herstellung der Mischungen aufgeteilt, und zwar in folgende Anteile: Mischung A: 25-40 %, Mischung B: 14-36 %, Mischung C: 15-40 %; ggf. kann ein Anteil zwischen 0 und 18 % zum Spülen der zur Herstellung der verschiedenen Mischungen verwendeten Behälter dienen.

Die Herstellung der Mischungen erfolgt in sterilen Gefäßen, die mit Rührer und ggf. Homogenisator ausgerüstet sind. Für den letzten Schritt ist ein temperierbarer und evakuierbarer Reaktor, ausgerüstet mit wirksamem Rührer vorteilhaft.

Wenn Wirkstoffe in den Gel-Emulsionen enthalten sind, werden diese in der Ölphase gelöst, sofern sie wasserunlöslich oder hydrolyseempfindlich sind. Die Lösung der Wirkstoffe in der Ölphase wird dann verarbeitet wie die Ölphase ohne Wirkstoffgehalt ("Mischung B").

In den folgenden Tabellen sind Beispiele mit verschiedenen Mischungsvarianten angegeben. Sie erläutern die Erfindung, sollen sie jedoch nicht beschränken. Es handelt sich immer um Emulsionen öliger Substanzen oder Lösungen in Hydrogelen (Basis Wasser). Viskositätsmessungen wurden mit einem Brookfield-Viskosimeter vorgenommen (Typ LV, Spindel 3, 1,5 U/min, 25°C, Messung der "Restviskosität": Spindel 30, Small Sample Adapter SC4-18/13R, 30 U/min, 25°C).

### Formulierungsbeispiele

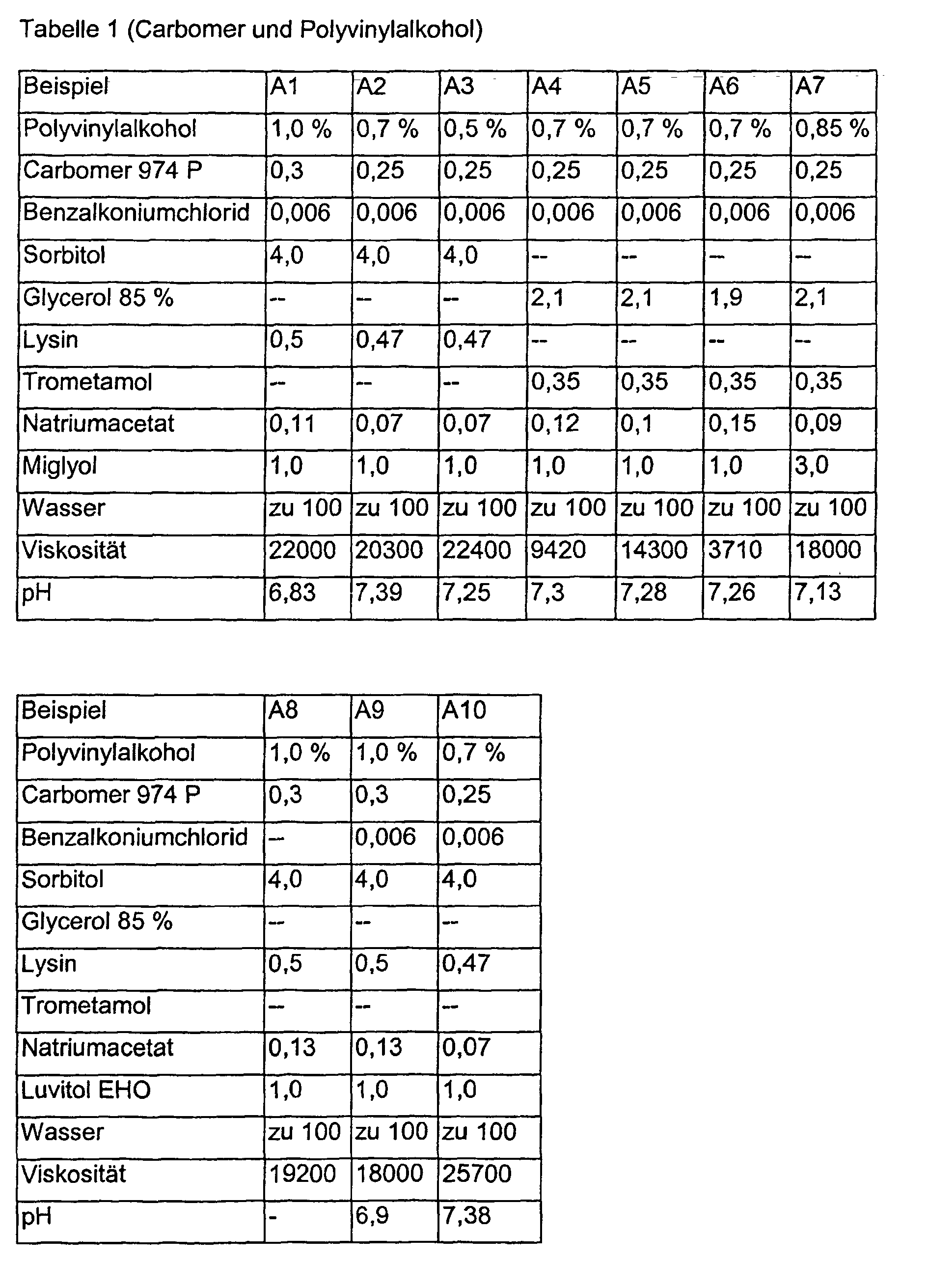

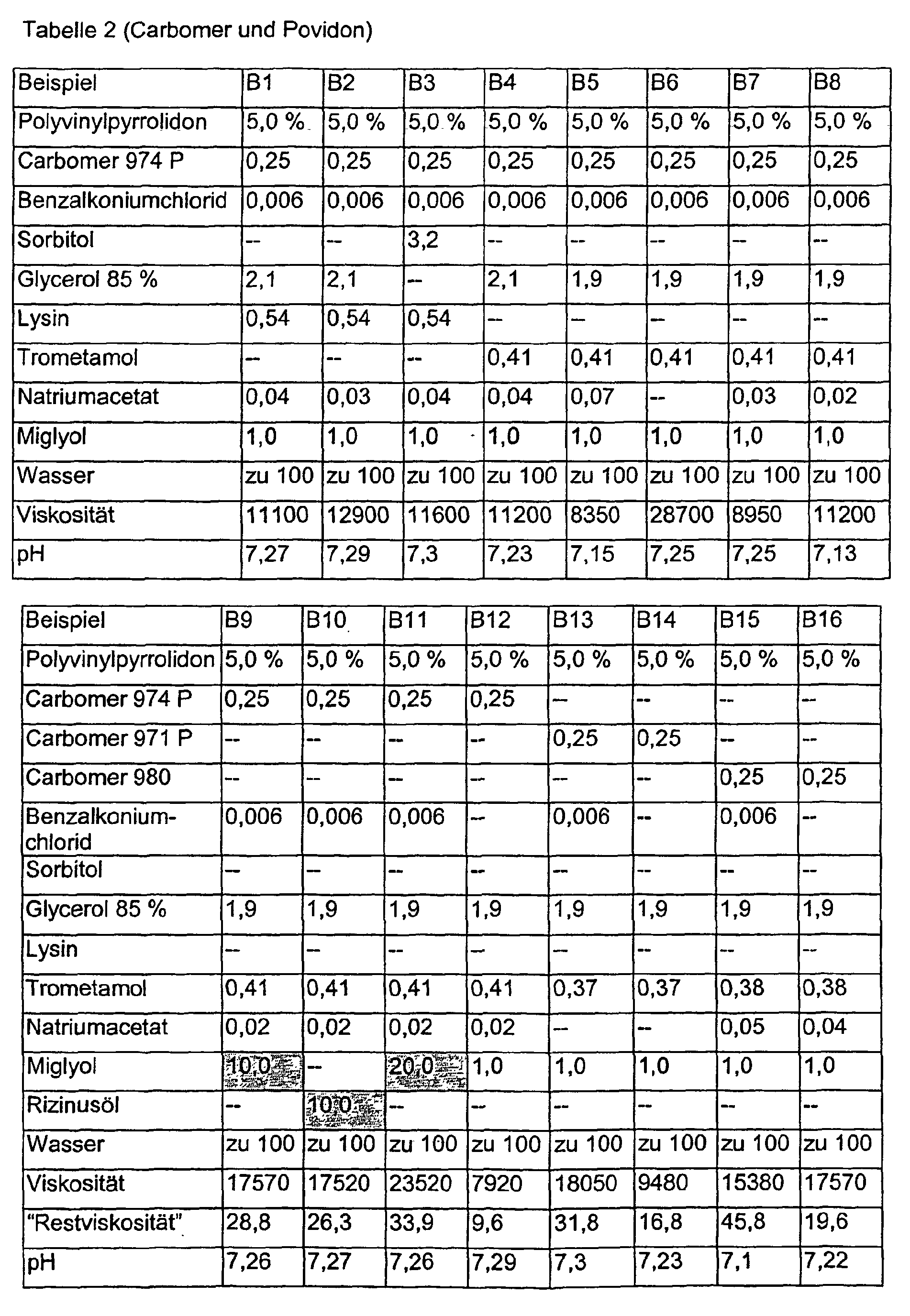

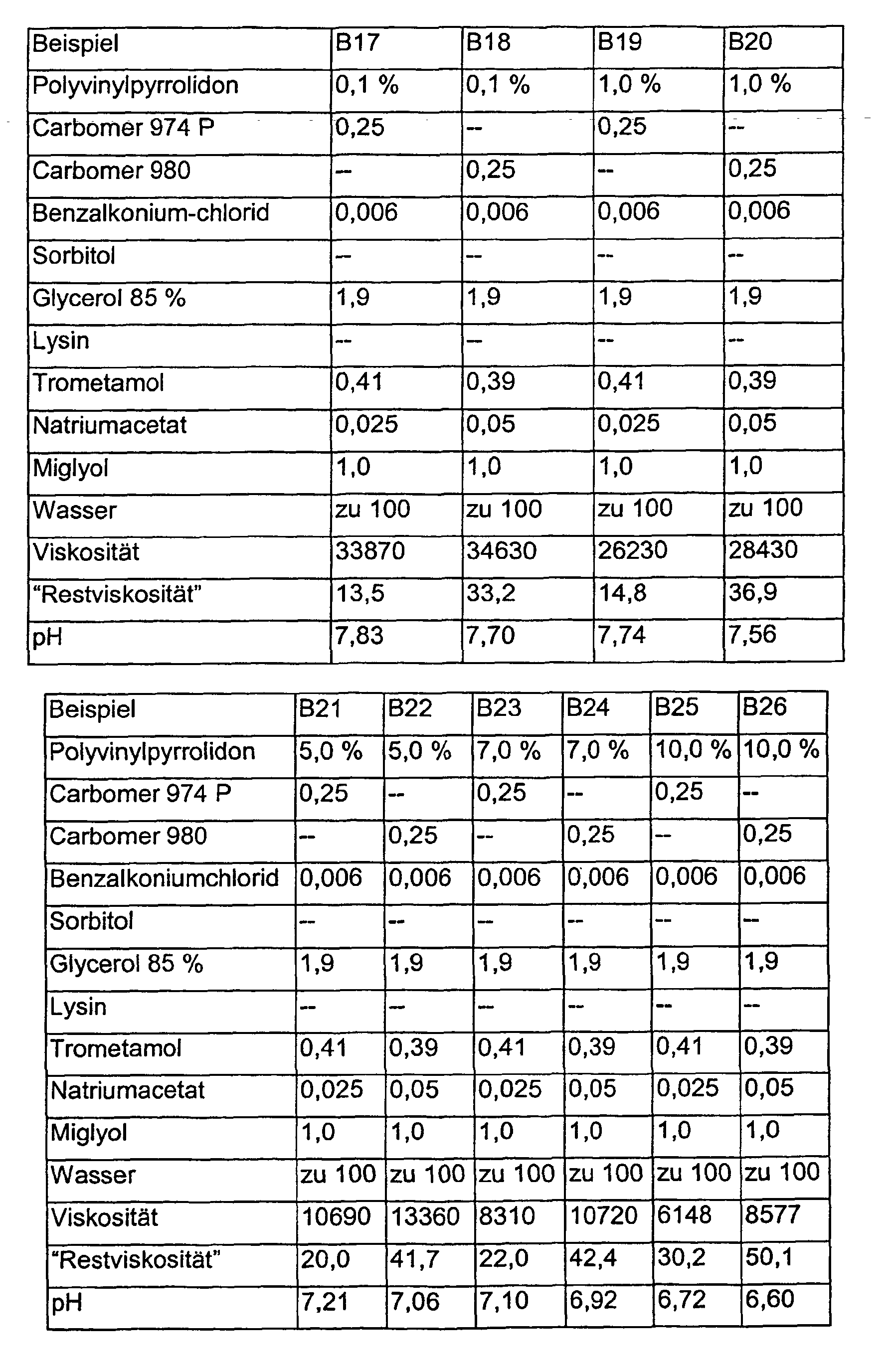

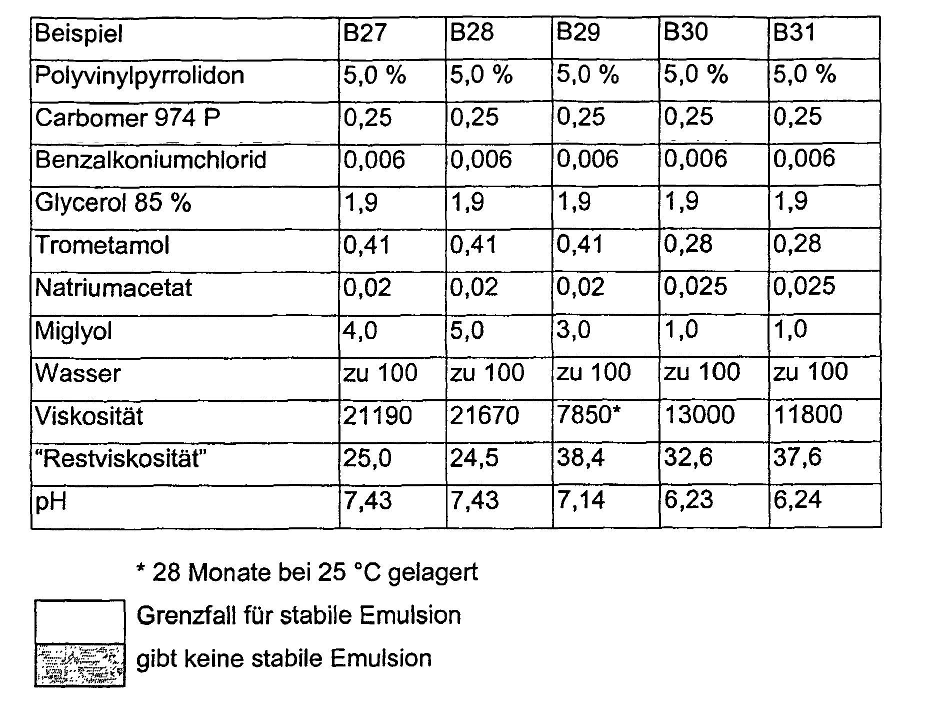

**Tabelle 3 (Carbomer und Dextran oder Hydroxymethylpropylcellulose)**

| Beispiel | D | H |
|---|---|---|
| Dextran | 2,0% | -- |
| Hydroxymethylpropylcellulose | -- | 0.2 % |
| Carbomer 974 P | 0,25 | 0,25 |
| Benzalkoniumchlorid | 0,006 | 0,006 |
| Sorbitol | -- | -- |
| Glycerol 85 % | 1,9 | 1,9 |
| Lysin | -- | -- |
| Trometamol | 0,35 | 0,35 |
| Natriumacetat | 0,1 | 0,12 |
| Miglyol | 1,0 | 1,0 |
| Wasser | zu 100 | zu 100 |
| Viskosität | 14600 | 14400 |
| pH | 7,14 | 7,14 |

**Tabelle 4 (Wirkstoffhaltige Gel-Emulsionen)**

| Beispiel | E1 | E2 | E3 | E4 | E5 |
|---|---|---|---|---|---|
| Polyvinylpyrrolidon | 5,0 % | 5,0 % | 5,0 % | 5,0 % | 5,0 % |
| Carbomer 974 P | 0,25 | 0,25 | 0,25 | 0,25 | 0,25 |
| Benzalkoniumchlorid | 0,006 | 0,006 | 0,006 | 0,006 | 0,006 |
| Glycerol 85 % | 1,9 | 1,9 | 1,9 | 1,9 | 1,9 |
| Trometamol | 0,41 | 0,41 | 0,41 | 0,41 | 0,41 |
| Natriumacetat | 0,025 | 0,025 | 0,025 | 0,025 | 0,02 |
| Miglyol | 1,0 | 2,0 | -- | 1,0 | 3,0 |
| Cholesterol | 0,02 | 0,04 | -- | -- | -- |
| Estradiol-Hemihydrat | -- | -- | 0,025 -- | | -- |
| Testosteronpropionat | -- | -- | -- | 0,025 | -- |
| Rizinusöl | -- | -- | 2,0 | -- | -- |
| Cyclosporin A | -- | -- | -- | -- | 0,2 |
| Wasser | zu 100 | zu 100 | zu 100 | zu 100 | zu 100 |
| Viskosität | 12200 | 12600 | 13400 | 11800 | |
| pH | 7,18 | 7,17 | - | 7,21 | 7,14 |

## Patentansprüche

1. Tropfbare Gel-Emulsion, insbesondere zur Anwendung am Auge, enthaltend ein Polymergemisch von Polyacrylat/Polyvinylalkohol, Polyacrylat/Polyvinylpyrrolidon, Polyacrylat/Dextran, oder Polyacrylat/Cellulosederivat, und eine Ölphase.

2. Gel-Emulsion nach Anspruch 1, **dadurch gekennzeichnet, dass** sie ein Polyacrylat mit einem Molekulargewicht zwischen 1.000.000 und 4.000.000 enthält.

3. Gel-Emulsion nach Anspruch 1 oder 2, **dadurch gekennzeichnet, dass** sie Salze zur Beeinflussung der Ausgangsviskosität enthält.

4. Gel-Emulsion nach Anspruch 3, **dadurch gekennzeichnet, dass** sie Natriumacetat enthält.

5. Gel-Emulsion nach einem der vorangehenden Ansprüche, **dadurch gekennzeichnet, dass** sie ein Polymergemisch von Polyacrylat/Polyvinylpyrrolidon enthält.

6. Gel-Emulsion nach einem der vorangehenden Ansprüche, **dadurch gekennzeichnet, dass** das Polyacrylat in einer Menge von 0,05 bis 3% und Polyvinylpyrrolidon in einer Menge von 0,05 bis 10% vorliegt.

7. Gel-Emulsion nach Anspruch 6, **dadurch gekennzeichnet, dass** das Polyacrylat in einer Menge von 0,05 bis 1% vorliegt.

8. Gel-Emulsion nach Anspruch 6 oder 7, **dadurch gekennzeichnet, dass** Polyvinylpyrrolidon in einer Menge von 1 bis 7% vorliegt.

9. Gel-Emulsion nach einem der Ansprüche 1 bis 4, **dadurch gekennzeichnet, dass** sie ein Polymergemisch von Polyacrylat/Hydroxypropylmethylcellulose enthält.

10. Gel-Emulsion nach einem der Ansprüche 1 bis 4, **dadurch gekennzeichnet, dass** sie ein Polymergemisch von Polyacrylat/Polyvinylalkohol enthält.

11. Gel-Emulsion nach einem der Ansprüche 1 bis 4, **dadurch gekennzeichnet, dass** sie ein Polymergemisch von Polyacrylat/Dextran enthält.

12. Gel-Emulsion nach einem der vorangehenden Ansprüche, **dadurch gekennzeichnet, dass** sie als übliche Zusätze Glycerol oder Sorbitol oder Mannitol enthält.

13. Gel-Emulsion nach einem der vorangehenden Ansprüche, **dadurch gekennzeichnet, dass** sie ein Konservierungsmittel enthält.

14. Gel-Emulsion nach Anspruch 13, **dadurch gekennzeichnet, dass** sie als Konservierungsmittel Benzalkoniumchlorid enthält.

15. Gel-Emulsion nach einem der vorangehenden Ansprüche, **dadurch gekennzeichnet, dass** sie eine Base enthält.

16. Gel-Emulsion nach Anspruch 15, **dadurch gekennzeichnet, dass** sie als Base Trometamol oder Lysin enthält.

17. Gel-Emulsion nach einem der vorangehenden Ansprüche, **dadurch gekennzeichnet, dass** sie einen oder mehrere Wirkstoffe aus in der Ophthalmologie verwendeten Wirkstoffklassen enthält.

18. Gel-Emulsion nach Anspruch 17, **dadurch gekennzeichnet, dass** sie als Wirkstoff Estradiol oder Cyclosporin A enthält.

19. Verfahren zur Herstellung einer Gel-Emulsion nach einem der Ansprüche 1 bis 18, **dadurch gekennzeichnet, dass** man
a) eine wäßrige Dispersion des Polyacrylats vorgibt, die das Isotonans und gegebenenfalls Konservierungsmittel enthält, und anschließend ein Gel herstellt gegebenenfalls unter Zugabe von Base und Salzen,
b) in einer wäßrigen Lösung von Polyvinylalkohol, Polyvinylpyrrolidon, Dextran oder einem Cellulosederivat, die gegebenenfalls das Konservierungsmittel oder einen Teil davon enthalten kann, die Ölphase mit einem Homogenisator feinst verteilt, und
c) die gemäß b) erhaltene Mischung in das gemäß a) erhaltene Gel durch Rühren homogen einarbeitet.

20. Verfahren gemäß Anspruch 19, **dadurch gekennzeichnet, dass** die gemäß b) eingesetzte Ölphase einen oder mehrere Wirkstoffe aus in der Ophthalmologie verwendeten Wirkstoffklassen enthält.

21. Verwendung einer Gel-Emulsion, die ein Polymergemisch von Polyacrylat/Potyvinylalkohol, Polyacrylat/Polyvinylpyrrolidon, Polyacrylat/Dextran oder Polyacrylat/Cellulosederivat und eine Ölphase und gegebenenfalls einen oder mehrere Wirkstoffe aus in der Ophthalmologie verwendeten Wirkstoffklasse enthält, zur Herstellung eines Arzneimittels zur Anwendung am Auge.

## Revendications

1. Emulsion de gel pouvant être appliquée en gouttes, en particulier destinée à une utilisation dans les yeux, contenant un mélange de polymères de polyacrylate/poly(alcool vinylique), polyacrylate/polyvinylpyrrolidone, polyacrylate/dextrane ou polyacrylate/dérivé de cellulose et une phase huileuse.

2. Emulsion de gel selon la revendication 1, **caractérisée en ce qu'**elle contient un polyacrylate présentant un poids moléculaire entre 1 000 000 et 4 000 000.

3. Emulsion de gel selon la revendication 1 ou 2, **caractérisée en ce qu'**elle contient des sels pour influencer la viscosité de départ.

4. Emulsion de gel selon la revendication 3, **caractérisée en ce qu'**elle contient de l'acétate de sodium.

5. Emulsion de gel selon l'une quelconque des revendications précédentes, **caractérisée en ce qu'**elle contient un mélange de polymères de polyacrylate/polyvinylpyrrolidone.

6. Emulsion de gel selon l'une quelconque des revendications précédentes, **caractérisée en ce que** le polyacrylate est présent en une quantité de 0,05 à 3% et la polyvinylpyrrolidone en une quantité de 0,05 à 10%.

7. Emulsion de gel selon la revendication 6, **caractérisée en ce que** le polyacrylate est présent en une quantité de 0,05 à 1%.

8. Emulsion de gel selon la revendication 6 ou 7, **caractérisée en ce que** la polyvinylpyrrolidone est présente en une quantité de 1 à 7%.

9. Emulsion de gel selon l'une quelconque des revendications 1 à 4, **caractérisée en ce qu'**elle contient un mélange de polymères de polyacrylate/hydroxypropylméthylcellulose.

10. Emulsion de gel selon l'une quelconque des revendications 1 à 4, **caractérisée en ce qu'**elle contient un mélange de polymères de polyacrylate/poly(alcool vinylique).

11. Emulsion de gel selon l'une quelconque des revendications 1 à 4, **caractérisée en ce qu'**elle contient un mélange de polymères de polyacrylate/dextrane.

12. Emulsion de gel selon l'une quelconque des revendications précédentes, **caractérisée en ce qu'**elle contient comme additifs usuels du glycérol ou du sorbitol ou du mannitol.

13. Emulsion de gel selon l'une quelconque des revendications précédentes, **caractérisée en ce qu'**elle contient un conservateur.

14. Emulsion de gel selon la revendication 13, **caractérisée en ce qu'**elle contient, comme conservateur, du chlorure de benzalconium.

15. Emulsion de gel selon l'une quelconque des revendications précédentes, **caractérisée en ce qu'**elle contient une base.

16. Emulsion de gel selon la revendication 15, **caractérisée en ce qu'**elle contient comme base du Trometamol ou de la lysine.

17. Emulsion de gel selon l'une quelconque des revendications précédentes, **caractérisée en ce qu'**elle contient une ou plusieurs substances actives des classes de substances actives utilisées en ophtalmologie.

18. Emulsion de gel selon la revendication 17, **caractérisée en ce qu'**elle contient comme substance active de l'estradiol ou de la cyclosporine A.

19. Procédé pour la préparation d'une émulsion de gel selon l'une quelconque des revendications 1 à 18, **caractérisé en ce qu'**on
a) dispose au préalable d'une dispersion aqueuse du polyacrylate, qui contient l'isotonan et le cas échéant un conservateur et on prépare ensuite un gel, le cas échéant avec addition de base et de sels,
b) répartit le plus finement possible dans une solution aqueuse de poly(alcool vinylique), de polyvinylpyrrolidone, de dextrane ou d'un dérivé de cellulose, qui peut contenir le cas échéant le conservateur ou une partie de celui-ci, la phase huileuse avec un homogénéisateur, et
c) incorpore de manière homogène par agitation le mélange obtenu selon b) dans le gel obtenu selon a).

20. Procédé selon la revendication 19, **caractérisé en ce que** la phase huileuse utilisée selon b) contient une ou plusieurs substances actives des classes de substances actives utilisées en ophtalmologie.

21. Utilisation d'une émulsion de gel qui contient un mélange de polymères de polyacrylate/poly(alcool vinylique), polyacrylate/polyvinylpyrrolidone, polyacrylate/dextrane ou polyacrylate/dérivé de cellulose et une phase huileuse et le cas échéant une ou plusieurs substances actives de classes de substances actives utilisées en ophtalmologie pour la préparation d'un médicament destiné à une utilisation dans les yeux.

## Claims

1. A drop-forming gel emulsion, in particular for applying to the eye, containing a polymer blend of polyacrylate/polyvinyl alcohol, polyacrylate/polyvinylpyrrolidone, polyacrylate/dextran or polyacrylate/cellulose derivative and an oil phase.

2. A gel emulsion according to claim 1, **characterised in that** it contains a polyacrylate with a molecular weight of between 1,000,000 and 4,000,000.

3. A gel emulsion according to claim 1 or claim 2, **characterised in that** it contains salts for influencing the initial viscosity.

4. A gel emulsion according to claim 3, **characterised in that** it contains sodium acetate.

5. A gel emulsion according to any one of the preceding claims, **characterised in that** it contains a polymer blend of polyacrylate/polyvinylpyrrolidone.

6. A gel emulsion according to any one of the preceding claims, **characterised in that** the polyacrylate is present in an amount of from 0.05 to 3% and polyvinylpyrrolidone is present in an amount of from 0.05 to 10%.

7. A gel emulsion according to claim 6, **characterised in that** the polyacrylate is present in an amount of from 0.05 to 1%.

8. A gel emulsion according to claim 6 or claim 7, **characterised in that** polyvinylpyrrolidone is present in an amount of from 1 to 7%.

9. A gel emulsion according to any one of claims 1 to 4, **characterised in that** it contains a polymer blend of polyacrylate/hydroxypropylmethylcellulose.

10. A gel emulsion according to any one of claims 1 to 4, **characterised in that** it contains a polymer blend of polyacrylate/polyvinyl alcohol.

11. A gel emulsion according to any one of claims 1 to 4, **characterised in that** it contains a polymer blend of polyacrylate/dextran.

12. A gel emulsion according to any one of the preceding claims, **characterised in that** it contains glycerol or sorbitol or mannitol as conventional additives.

13. A gel emulsion according to any one of the preceding claims, **characterised in that** it contains a preservative.

14. A gel emulsion according to claim 13, **characterised in that** it contains benzalkonium chloride as preservative.

15. A gel emulsion according to any one of the preceding claims, **characterised in that** it contains a base.

16. A gel emulsion according to claim 15, **characterised in that** it contains trometamol or lysine as base.

17. A gel emulsion according to any one of the preceding claims, **characterised in that** it contains one or more active ingredients from classes of active ingredient used in ophthalmology.

18. A gel emulsion according to claim 17, **characterised in that** it contains oestradiol or cyclosporin A as active ingredient.

19. A process for the production of a gel emulsion according to any one of claims 1 to 18, **characterised in that**
a) an aqueous dispersion of the polyacrylate, which contains the isotonicity agent and optionally the preservative, is initially introduced and then a gel is produced, optionally with the addition of base and salts.
b) the oil phase is ultra-finely dispersed with a homogeniser in an aqueous solution of polyvinyl alcohol, polyvinylpyrrolidone, dextran or a cellulose derivative, which may optionally contain the preservative or part thereof, and
c) the blend obtained according to b) is homogeneously incorporated by stirring into the gel obtained according to a).

20. A process according to claim 19, **characterised in that** the oil phase used according to b) contains one or more active ingredients from classes of active ingredients used in ophthalmology.

21. Use of a gel emulsion containing a polymer blend of polyacrylate/polyvinyl alcohol, polyacrylate/polyvinylpyrrolidone, polyacrylate/dextran or polyacrylate/cellulose derivative and an oil phase and optionally one or more active ingredients from classes of active ingredients used in ophthalmology to produce a pharmaceutical preparation for applying to the eye.
